# EUROPEAN PATENT APPLICATION

(11) **EP 4 230 242 A1**
(43) Date of publication of application: **23.08.2023**
(21) Application number: 22305189.7
(22) Date of filing: 21.02.2022
(51) Int. Cl.: A61M 5/34, A61M 39/10, A61M 39/20, A61M 5/31

(54) **ADAPTOR FOR CONNECTING A CONNECTOR TO A DISTAL TIP OF A MEDICAL CONTAINER, TIP CAP ASSEMBLY AND DRUG DELIVERY DEVICE FOR ENGAGING SAID ADAPTOR, AND METHOD FOR MANUFACTURING SAID ADAPTOR**

(71) Applicant: Becton Dickinson France, 38800 Le Pont-de-Claix (FR)
(72) Inventor: JAMON, Rémi, 38410 VAULNAVEYS LE HAUT (FR); NICOLAS, Maxime, 38000 GRENOBLE (FR); Lubin, DURAND, 07170 MIRABEL (FR)
(74) Representative: Germain Maureau

(57) **Abstract**

This adaptor (1) comprises a tubular lateral wall (2) extending around a longitudinal axis (A), the tubular lateral wall (2) defining an opened proximal end (22) for receiving the distal tip (102) and an opposite opened distal end (23) for receiving the connector. An inner ring (3) located at the proximal end (22) secures the adaptor (1) to the distal tip (102) of the medical container (100). Two inner bulges (4) inwardly protruding from the tubular lateral wall (2) define a unique inner cavity (40) for guiding translation of the connector inside the adaptor (1) along the longitudinal axis (A). A distal end (23) of the inner bulge (4) defines an index opening (42) for orienting the connector with regard to the adaptor (1) in a first angular position in which the connector is allowed to enter the inner cavity (40). The inner bulge (4) includes a first blocking surface (44) for blocking rotation of the connector in a first direction during insertion of the connector in the inner cavity (40). The adaptor further comprises two locking slots (6) located proximally to the inner cavity (40) and distally to the inner ring (3) for allowing the connector in an inserted position to rotate around the longitudinal axis (A) in a second direction opposite said first direction, such that outer wings (204) of the connector engage the locking slots (6). The locking slots (6) include a mechanical stop (62) for stopping rotation of the connector in a second angular position in which a proximal abutment surface (60) of the locking slots (6) prevents the connector from moving back in the distal direction.

## Description

The present invention relates to an adaptor for allowing assembly of a connector, such as a needle hub or tip cap assembly, to a medical container, a drug delivery device comprising said adaptor, a tip cap assembly configured to engage the adaptor in order to seal a distal tip of said medical container, and a method for manufacturing the adaptor.

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction of injection, with respect to an adaptor or a medical container of the invention, and the "proximal direction" is to be understood as meaning the opposite direction to said direction of injection, that is to say the direction towards the user's hand holding a medical container as for an injection operation.

Basically, medical containers, such as for example pre-fillable or prefilled syringes, are preferably made of glass for its high chemical passivity, its low gas permeability and high transparency, which allows an extended storage and an easy inspection.

Medical containers usually comprise a barrel defining a reservoir for containing a medical product. The container has a distal end in the form of a longitudinal tip defining an axial passageway in fluid communication with the reservoir so as to allow the medical product to flow outside the reservoir. However, this longitudinal tip does not allow parenteral administration by itself and must either comprise a staked needle or an adaptor allowing the connection of the syringe to a connector such as a needle hub. The adaptor is typically screwed, glued or snap-fitted to the longitudinal tip of the syringe. This connector is typically screwed into the adaptor and engages the distal end of the container to establish a fluid path between the reservoir and the connector.

It is important that the connector be properly screwed into the adaptor. The end user has to apply a sufficient torque in order to get a proper fitting between the connector and the distal end of the container. However, it may be difficult for the end user to know exactly when the proper fitting is achieved. Under-screwing may cause ejection of the connector or cause a leakage during transfer of a fluid from the container to the connector, while over-screwing may alter the connection between the adaptor and the longitudinal tip and thus cause the adaptor to undesirably rotate or tilt with respect to the longitudinal tip.

There is therefore a need to ease the connection between a connector and an adaptor. There is a need for a user to know when the connection between a connector and the longitudinal tip of a medical container is properly achieved.

Besides, adaptors are usually made of plastic material by injection molding. There is also a need for simplifying the molding process and reduce the manufacturing costs.

An aspect of the invention is an adaptor for connecting a connector comprising two outer wings to a distal tip of a medical container, the adaptor comprising:
a tubular lateral wall extending around a longitudinal axis A, the tubular lateral wall defining an opened proximal end for receiving the distal tip and an opposite opened distal end for receiving the connector,
an inner ring located at the proximal end for securing the adaptor to the distal tip of the medical container,
two inner bulges inwardly protruding from the tubular lateral wall, the two bulges defining a unique inner cavity for guiding translation of the connector inside the adaptor along the longitudinal axis A, a distal end of the inner bulges defining an index opening for orientating the connector with regard to the adaptor in a first angular position in which the connector is allowed to enter the inner cavity, the inner bulges including a first blocking surface for blocking rotation of the connector in a first rotational direction during insertion of the connector in the inner cavity,
two locking slots located proximally to the inner cavity and distally to the inner ring for allowing the connector in an inserted position to rotate around the longitudinal axis A in a second rotational direction opposite said first rotational direction, such that each of the outer wings of the connector engage one of the locking slots, the locking slots including a mechanical stop for stopping rotation of the connector in a second angular position and an abutment surface for preventing the connector from moving back in the distal direction.

The adaptor of the invention thus permits to establish a safe connection between a connector (needle hub or tip cap assembly) and the distal tip of a medical container. The connection movement merely consists in an axial translation followed by a rotation around the longitudinal axis A. There is accordingly a lower risk, or even no risk, of over- or under-screwing.

The adaptor indeed does not require use of an internal thread, thereby also allowing to simplify the molding process. The mold is simpler and thus less expensive. Moreover, the adaptor is easier and faster molded by injection molding. There is no need to use a rotating core pin, as usually used for molding adaptors having an internal thread, thereby savins costs.

The adaptor further provides haptic or tactile feedback that the connection is safe and complete. Indeed, thanks to the mechanical stop, the end-user can feel that the connection is complete. Risks of leakage or inadvertant disconnection are thus avoided.

The adaptor may further comprise some or all of the advantageous features below.

In an embodiment, the inner ring has inwardly protruding lugs for abutting against the distal tip. This improves the attachment of the adaptor on the distal tip. Risks of leakage or of disconnection are further limited. A distal end of the lugs is axially positioned proximal to the abutment surface of the locking slots so that the lugs do not interfere with the connector.

In an embodiment, the inner ring includes a distal side having a tapered or frustroconical shape. This improves the connection of the adaptor to the distal tip of the syringe, especially when a radially inwardly extending groove is present on the external surface of the distal tip: the tapered or frustroconical shape of the inner ring enables easier introduction of the adaptor around the distal tip, and allows deflection of the lugs when in contact of the groove.

In an embodiment, the locking slots are through-openings extending through the lateral wall of the adaptor. Thus, the user can see whether the outer wing of the connector is engaged or not in the locking slot. This provides the user with a visual indication that the connection is completed. This also simplify the molding process.

In an embodiment, the first angular position and the second angular position are separated by an angle comprised between 40° and 90°, preferably between 50° and 80°, and even more preferably by an angle of 65°.

In an embodiment, the inner bulges include a second blocking surface for blocking rotation of the connector in the second rotational direction as long as the connector is being slid inside the adaptor, the second blocking surface being configured to allow rotation of the connector in the second rotational direction when the connector is in a fully inserted position. The second blocking surface for instance ends at the locking slot or distal to the locking slot. Therefore, the connector cannot rotate inside the inner cavity as long as the connector is not fully inserted. The first blocking surface may extend from the index opening to the inner ring, such that the first blocking surface blocks rotation of the connector even when the connector is in the inserted position. The second blocking surface may extend from the index opening to the locking slot (and more specifically to a distal wall of the locking slot). The second blocking surface is thus axially shorter than the first blocking surface. Unlike the first blocking surface, the second blocking surface does not extend up to the inner ring. The connector in the inserted position can thus engage the locking slot by rotation in the second rotational direction only. The first and second blocking surfaces may longitudinally extend parallel to the longitudinal axis A.

The inner cavity may be defined by an inner wall parallel to the longitudinal axis A. The bulges have a cylindrical portion between the first stop and the second blocking surfaces. The inner cavity is smooth. That is, the inner wall delimiting the inner cavity is devoid of any internal thread or radial protrusion.

In an embodiment, the adaptor includes a tapered portion extending between the opened distal end of the adaptor and the inner cavity. This permits to center the connector with regard to the longitudinal axis A. The end-user then just needs to rotate the connector towards the first angular position so that the connector can enter the index opening.

In an embodiment, the inner bulges have a flat distal surface. This distal surface, which is located at the distal end of the inner bulges, is orthogonal to the longitudinal axis A. The tapered portion extends between the distal face of the adaptor and the flat distal surface of the inner bulges.

In an embodiment, the two inner bulges and the two locking slots are diametrically opposite.

Another aspect of the invention is a tip cap assembly configured to be connected to the above described adaptor, wherein the tip cap assembly has two outwardly protruding wings configured to engage the locking slots of the adaptor for removably securing the tip cap assembly to the adaptor.

Possibly, the outwardly protruding wings have a guiding surface, such as a chamfer or a frustroconical shape on a distal side thereof, so as to ease insertion of the outwardly protruding wings into the locking slots of the adaptor.

Another aspect of the invention is a drug delivery device including a medical container having a distal tip longitudinally extending from a barrel defining a reservoir for a medical product, the above-described adaptor configured to be mounted onto the distal tip, and a connector, such as a needle hub or a tip cap assembly, configured to be removably secured to the distal tip by means of said adaptor.

In an embodiment, the connector and the inner cavity of the adaptor are complementarily shaped. The connector thus cannot rotate or tilt during insertion inside the adaptor. This improves positioning of the connector relative to the adaptor and to the distal tip, thereby reducing the risks of leakage.

In an embodiment, the proximal abutment surface of each of the locking slots abuts against at least 50%, preferably between 50% and 90%, of a distal side of the corresponding outwardly protruding wing when the connector is in the second angular position.

Another aspect of the invention is a method for manufacturing the above-described adaptor. The method is devoid of the use of a rotating core pin. Indeed, the adaptor does not need an internal thread for securing the connector. This provides an improved and cost-effective molding process.

The invention and the advantages arising therefrom will clearly emerge from the detailed description that is given below with reference to the appended drawings as follows:
- Figures 1A, 1B, 1C and 1D are, respectively, a perspective view, a cross-section view, a partial side view and a top view of an adaptor according to an embodiment of the invention,
- Figure 2 is a perspective view of medical container engaged with an adaptor according to an embodiment of the invention,
- Figures 3A, 3B and 3C are, respectively, a perspective view, a partial perspective view and a cross-section view of an adaptor according to an embodiment of the invention,
- Figures 4A and 4B are, respectively, a perspective view and a bottom view of a needle hub designed to engage an adaptor according to an embodiment of the invention,
- Figure 5A is a side cross-section view of a medical container and a needle hub engaged with an adaptor according to an embodiment of the invention,
- Figure 5B is a detail of Figure 5A,
- Figures 6A and 6B are, respectively, a perspective view and a bottom view of the outer cap of a tip cap assembly designed to engage an adaptor according to an embodiment of the invention,
- Figures 7A and 7B are, respectively, a cross-section view and a perspective view of a medical container and a tip cap assembly engaged with an adaptor according to an embodiment of the invention,
- Figures 8A to 8C are side views illustrating the steps of engaging a needle hub with a medical container provided with an adaptor according to an embodiment of the invention,
- Figures 9A and 9B are top views of a medical container and a needle hub engaged with an adaptor according to an embodiment of the invention, wherein the needle hub is respectively in an unlocked position and a locked position.

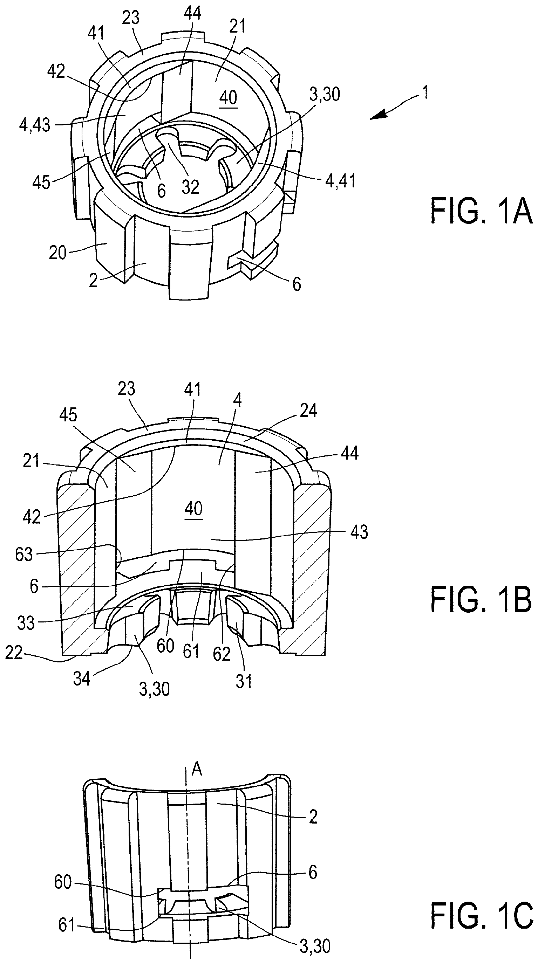

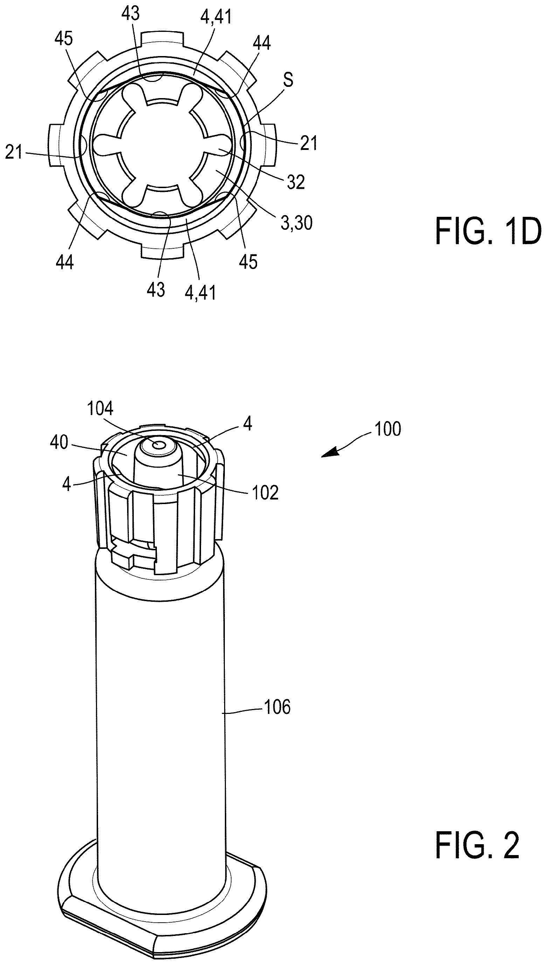

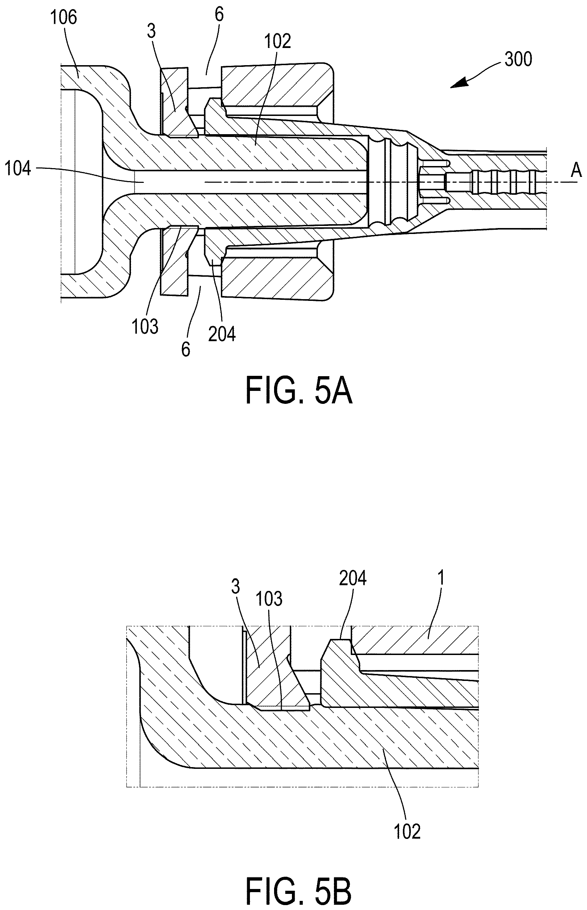

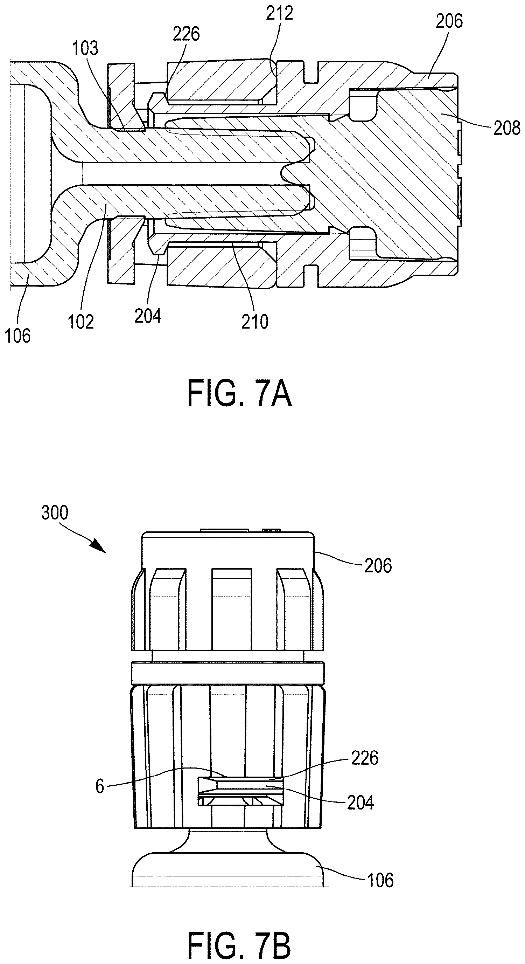

With reference to Figure 1A is shown an adaptor 1 according to an embodiment of the invention. As shown in Figure 2, the adaptor 1 is configured to be secured to a distal tip 102 of a medical container 100. Besides, the adaptor 1 is configured to receive a connector, such as needle hub 200 (Figure 5A) or a tip cap assembly 202 (Figure 7A), and to secure the connector onto the distal tip 102 of the medical container 100.

As illustrated in Figures 1A, 1B and 1C, the adaptor 1 includes a tubular lateral wall 2 extending around a longitudinal axis A. The lateral wall 2 of the adaptor 1 may have a cylindrical shape. On an outer surface thereof, axial ribs 20 may be arranged for allowing an end-user to grasp the adaptor 1. The lateral wall 2 has an inner surface which has two diametrically opposite cylindrical portions 21. The tubular lateral wall 2 leads to an opened proximal end 22 configured to receive the distal tip 102 of the medical container 100 and to an opposite opened distal end 23 configured to receive the connector.

The proximal end 22 of the adaptor 1 has an inner ring 3 for securing the adaptor 1 onto the distal tip 102 of the medical container 100. As visible on Figures 5A and 5B, the distal tip 102 may include a groove 103 and the inner ring 3 ring may be configured to engage the groove 103 of the distal tip 102 in order to secure the adaptor 1 on the distal tip 102.

As shown in Figures 1A and 1D, the inner ring 3 may comprise inwardly radially protruding lugs 30, which may be regularly distributed in a circumferential direction. The lugs 30 include a free end 31, which may have a cylindrical shape, for abutting against the outer surface of the distal tip 102, or the outer surface of the groove 103 present on the distal tip 102. The lugs 30 may be separated by opened radial windows 32 for allowing the lugs 30 to slightly deform when the adaptor 1 is mounted onto the distal tip 102. As illustrated in Figures 1B, 5A or 7A, a distal side 33 of the inner ring 3 may have a distally tapering shape or frustoconical shape, whereas a proximal side 34 of the inner ring 3 may be orthogonal to the longitudinal axis A.

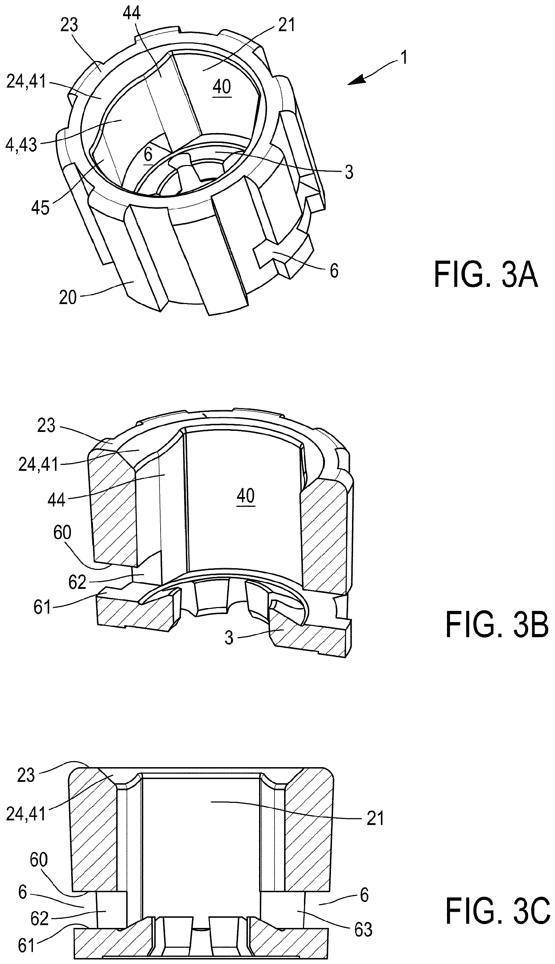

With reference to Figure 1A, two diametrically opposite inner bulges 4 protrude from the inner surface of the lateral wall 2. The inner bulges 4 define between them an inner cavity 40 for guiding insertion of the connector in the proximal direction inside the adaptor 1. The inner bulges 4 have a distal end 41, which may have a flat surface (orthogonal to the longitudinal axis A) for allowing positioning of the connector relative to the adaptor 1 before insertion. A tapered portion 24, having for example a frustroconical shape, may extend between the distal end 23 of the adaptor 1 and the distal end 41 of the inner cavity 40 for centering the connector. In an alternative embodiment illustrated on Figures 3A to 3C, the distal end 41 of the inner bulges 4 is tapered and thus forms the tapered portion 24.

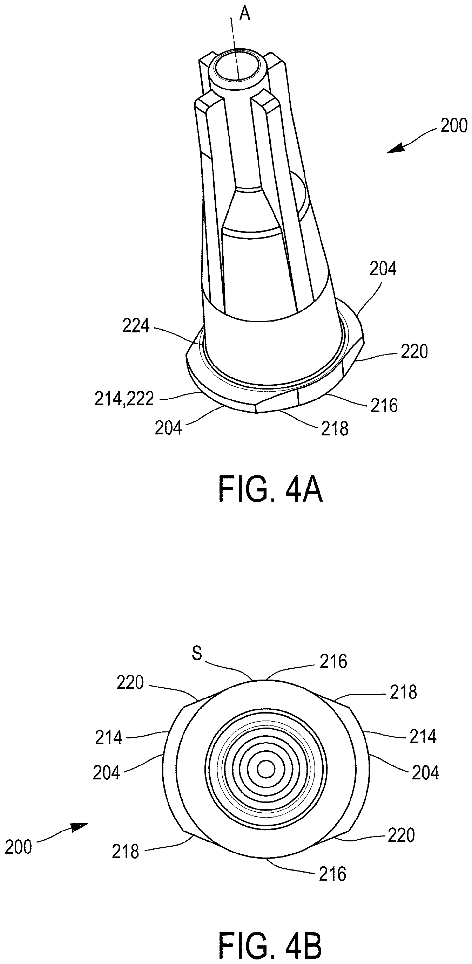

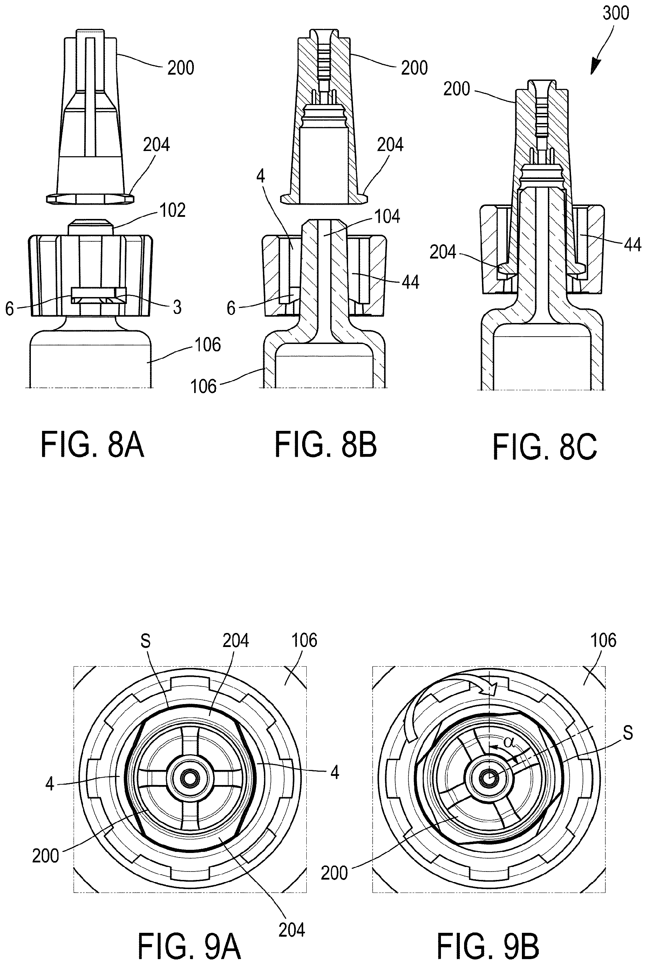

The distal ends 41 of the inner bulges 4 further define an index opening 42. The index opening 42 is the entry of the inner cavity 40, which makes only one possibility for inserting the connector into the adaptor 1. The index opening 42 allows insertion of the connector inside the inner cavity 40 only if the connector is rotationally positioned in a first predetermined angular position (Figure 9A). That is, the index opening 42 permits entry of the connector in the inner cavity 40 when the connector is in the first angular position, and prevents the connector from entering the inner cavity 40 as long as the connector is in an angular position different from said first angular position. To that end, the index opening 42 and the connector, such as the needle hub 200 or the tip cap assembly 202, have the same cross-section shape S, as can be seen in Figures 1D, 4B and 6B. More specifically, the inner bulges 4 may have a cylindrical surface 43, a first flat surface 44 at one side of the cylindrical surface 43 and a second flat surface 45 at the other side of the cylindrical surface 43. The cylindrical surfaces 43 of the inner bulges 4, the first flat surfaces, the second flat surfaces and the cylindrical portions 21 of the lateral wall 2 delimit the inner cavity 40 and thus form the predetermined cross-section shape S.

The inner bulges 4 may be configured to prevent rotation of the connector as long as the connector is not fully inserted into the adaptor 1, but to allow rotation of the connector once the connector is fully inserted into the adaptor 1, meaning that the connector may not be more distally inserted. To that end, the inner bulges 4 have a first blocking surface 44, which is the first flat surface, for blocking rotation of the connector in a first rotational direction, preferably the counterclockwise direction, and a second blocking surface 45, which is the second flat surface, for blocking rotation of the connector in an opposite second rotational direction, preferably the clockwise direction.

As shown in Figure 1B, the first blocking surface 44 may axially extend from the distal end 23 of the inner bulge 4 to the inner ring 3. As a result, the connector, in the proximal-most position illustrated in Figure 5A, cannot rotate in the first rotational direction. The proximal-most position corresponds to a fully inserted position of the connector inside the adaptor 1, in which the connector cannot be slid any further in the proximal direction.

Unlike the first blocking surface 44, the second blocking surface 45 does not axially extend until the inner ring 3. As illustrated in Figure 1B, the second blocking surface 45 may axially extend from the distal end 23 of the inner bulge 4 to a locking slot 6 extending between a proximal end of the second blocking surface 45 and the inner ring 3. The proximal end of the second blocking surface 45 is thus axially located distal to the inner ring 3. The second blocking surface 45 is accordingly axially shorter than the first blocking surface 44. The second blocking surface 45 allows the connector to engage the locking slot 6 by rotation in the second rotational direction.

It is contemplated that the inner cavity 40 of the adaptor 1 is devoid of any internal thread. The cylindrical portions 21 of the lateral wall 2, the cylindrical surfaces 43 of the inner bulges 4 and the first and second blocking surfaces 44, 45 all extend parallel to the longitudinal axis A. They are smooth and have no radial protrusions. As a result, the insertion of the connector inside the adaptor 1 is a translational movement along the longitudinal axis A without rotation.

Insertion of the connector into the adaptor 1 is limited by the conical contact between an inner surface of the connector and the outer surface of the distal tip 102. The proximal-most position (inserted position) may be the leakage limit position of the needle hub 200 as defined in the ISO 80369-7 (2016) (paragraphs 6.1 and 6.2).

The adaptor 1 further includes two diametrically opposite locking slots 6, which are preferably arranged at a proximal end 22 of the inner bulges 4. The locking slots 6 are configured to receive outer wings 204 of the connector when the connector is rotated in the second rotational direction (clockwise direction). The locking slots 6 longitudinally extend in a circumferential direction. Preferably, the locking slots 6 are through-openings radially extending from side to side of the lateral wall 2 of the adaptor 1. As a result, the user can see whether the connector is properly engaged or not in the locking slots 6.

The locking slots 6 may have four walls: an upper (distal) wall 60 and a lower (proximal) wall 61 which may be orthogonal to the longitudinal axis A, and two lateral sidewalls 62, 63. The distal wall 60, the proximal wall 61 and the first and second sidewalls 62, 63 preferably are flat surfaces.

The distal wall 60 of the locking slot 6 forms a proximal abutment surface configured to abut against a distal side of the outer wings 204 of the connector for preventing the connector to move back in the distal direction. The proximal abutment surface 60 is dimensioned to cover at least 50%, preferably between 50% and 90%, of the distal side surface of the corresponding outer wing 204. This distal wall 60 is always contacting the distal side surface of the corresponding outer wing 204 when the connector is rotated towards the second angular position.

A first one of the two sidewalls 62, 63 may be configured such that the outer wings 204 of the connector abut against the first sidewalls 62 of the locking slots 6 when the connector rotates in the second rotational direction, but do not abut against the second sidewalls 63 when the connector rotates back in the first rotational direction. The first sidewall 62 of the locking slot 6 is next to the first blocking surface 44 of the inner bulge 4, while the second sidewall 63 is next to the second blocking surface 45.

The first sidewall 62 of the locking slot 6 thus forms a mechanical stop for stopping rotation of the connector in a second predetermined angular position, when the connector is rotated in the second rotational direction. In said second angular position, connection between the connector and the adaptor 1 is complete. With reference to Figures 9A and 9B, the angle α defined between the first and second angular positions is comprised between [40°-90°], preferably between [50°-80°], and even more preferably by an angle of about 65°.

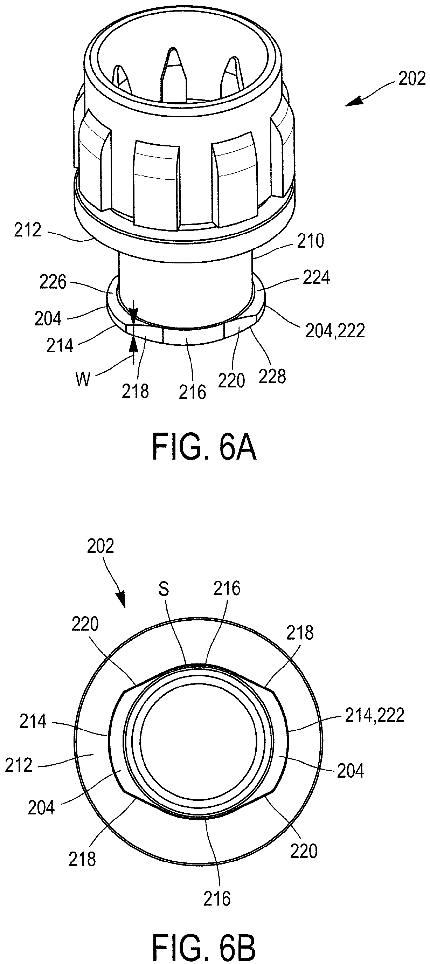

With reference to Figures 6A to 7B, the invention also relates to a tip cap assembly 202. The tip cap assembly 202 is configured to be secured to the adaptor 1 in order to seal an axial passageway 104 of the distal tip 102. The tip cap assembly 202 includes an outer cap 206, and an inner cap 208 arranged inside the outer cap 206. The outer cap 206 may be made of a rigid material and the inner cap 208 may be made of a resiliently deformable material. The outer cap 206 includes a skirt 210 proximally protruding from a shoulder 212. The shoulder 212 is configured to abut against the distal end 23 of the adaptor 1, while the skirt 210 is configured to engage the inner cavity 40.

The tip cap assembly 202 has two diametrically opposite outer wings 204 configured to engage the two locking slots 6 of the adaptor 1. The outer wings 204 are located at a proximal end 22 of the skirt 210. They extend orthogonal to the longitudinal axis A. The outer wings 204, the index opening 42 and the inner cavity 40 have the same cross-section shape S.

More specifically, the outer wings 204 define a lateral surface for guiding insertion of the connector inside the inner cavity 40 of the adaptor 1, and the lateral surface may include: two pairs of diametrically opposite cylindrical surfaces 214, 216 for cooperating, respectively, with the cylindrical portions 21 of the adaptor 1 and the cylindrical surfaces 43 of the inner bulges 4, and four abutment surfaces 218, 220, which may be flat and parallel to the longitudinal axis A. Two of the abutment surfaces 218 are configured to abut against the first blocking surfaces 44 of the inner bulges 4, while the two other abutment surfaces 220 are configured to abut against the second blocking surfaces 45 (during translation of the connector in the adaptor 1) and then against the first sidewall 62 of the locking slot 6 (when the connector is rotated from the first to the second angular position).

The outer wings 204 have an ouwtardly radially decreasing width w. As a result, a free end 222 of the outer wings 204 may be thinner than a base portion 224 of the outer wings 204. That is, the distal side of the outer wings 204 may include a guiding surface 226 having a chamfer or a frustoconical shape, to ease insertion of the outer wings 204 into the locking slots 6 of the adaptor 1. When engaged in the locking slots 6, the guiding surface 226 of the outer wings 204 abuts against the proximal abutment surface 60 of the adaptor 1 to block axial movement of the connector in the distal direction. Opposite the distal side and the guiding surface 226, the outer wings 204 have a proximal side 228.

It shoud be noted that the tip cap assembly 202 is devoid of any external thread. This simplifies the manufacturing process of the tip cap assembly 202.

With reference to Figures 7A-7B or 8A-8C, the invention also relates to a drug delivery device 300 including a medical container 100 and the above-described adaptor 1. The medical container 100 has a tubular barrel 106 defining a reservoir for a medical product and a longitudinally protruding distal tip 102 defining an axial passageway 104 in fluid communication with the reservoir. The outer surface of the distal tip 102 is preferably conical. The distal tip 102 may include a groove 103 for receiving the inner ring 3 of the adaptor 1 and secure the adaptor 1 to the distal tip 102. As illustrated in Figures 5A and 7A, the drug delivery device 300 may further include a needle hub 200 (Figure 5A) for fluidly connecting the axial passageway 104 of the distal tip 102 to a needle or IV catheter (not shown), or the above-described tip cap assembly 202 (Figure 7A) for sealing the axial passageway 104.

The operation of the drug delivery device 300 according to the invention will now be described below with reference to Figures 8A-8C and 9A-9B.

As illustrated in Figures 8A and 8B, the needle hub 200 (or tip cap assembly 202) must be firstly positioned in the first angular position. The first angular position permits entry of the connector inside the inner cavity 40 of the adaptor 1 via the index opening 42.

The needle hub 200 is proximally translated along the longitudinal axis A inside the inner cavity 40 of the adaptor 1 until the needle hub 200 is in the proximal-most position, i.e. in the fully inserted position (Figure 8C). The needle hub 200 has been guided by the inner bulges 4 of the adaptor 1 without rotating, such that the needle hub 200 is still in the first angular position (Figure 9A). At this stage, the cone-shaped needle hub 200 properly fits onto the complementarily-shaped distal tip 102 (Figure 8C), but the connection is not yet complete since the needle hub 200 can move back in the distal direction.

The user only needs to rotate the needle hub 200 in the second rotational direction (clockwise direction). This is possible because the second blocking surfaces 45 of the inner bulges 4 do not block rotation in the second rotational direction any longer. Besides, the outer wings 204 are now positioned at the locking slots 6. Rotation of the needle hub 200 in the second rotational direction accordingly causes the outer wings 204 to engage the locking slots 6. The outer wings 204 abut against the first sidewall 62 of the locking slots 6 (Figure 9B), thereby providing the user with a haptic feedback that the connection is complete. The distal side 226 of the outer wings 204 abuts against the upper (distal) wall 60 of the locking slots 6. The connector is thus axially blocked and removably secured to the distal tip 102 of the medical container 100.

To remove the connector, the user has to firstly turn the connector back in the first rotational direction until the outer wings 204 of the connector abut against the first blocking surfaces 44 of the inner bulges 4 and then slide the connector back in the distal direction so that the connector exits the inner cavity 40.

The invention also relates to a method for manufacturing the above-described adaptor 1. The method includes a step of forming the adaptor 1 by injection molding. The method is however devoid of the use of a rotating core pin because the adaptor 1 does not need an internal thread for securing the connector.

The invention accordingly permits to removably secure a connector to the distal tip 102 of a medical container 100 without risks of over- or under-screwing. The adaptor 1 is also easier to manufacture since the method for manufacturing this adaptor 1 does not require the use of a rotating core pin. The invention is thus a cost-effective solution to easily connect a needle hub 200 or tip cap assembly 202 to the distal tip 102 of a medical container 100 (axial translation followed by a limited rotation), while reliably preventing leakage between the distal tip 102 and said needle hub 200 or tip cap assembly 202.

## Claims

1. Adaptor (1) for connecting a connector comprising two outer wings (204) to a distal tip (102) of a medical container (100), the adaptor (1) comprising:
a tubular lateral wall (2) extending around a longitudinal axis (A), the tubular lateral wall (2) defining an opened proximal end (22) for receiving the distal tip (102) and an opposite opened distal end (23) for receiving the connector,
an inner ring (3) located at the proximal end (22) for securing the adaptor (1) to the distal tip (102) of the medical container (100),
two inner bulges (4) inwardly protruding from the tubular lateral wall (2), the two bulges (4) defining a unique inner cavity (40) for guiding translation of the connector inside the adaptor (1) along the longitudinal axis (A), a distal end (23) of the inner bulges (4) defining an index opening (42) for orientating the connector with regard to the adaptor (1) in a first angular position in which the connector is allowed to enter the inner cavity (40), the inner bulges (4) including a first blocking surface (44) for blocking rotation of the connector in a first rotational direction during insertion of the connector in the inner cavity (40),
two locking slots (6) located proximally to the inner cavity (40) and distally to the inner ring (3) for allowing the connector in an inserted position to rotate around the longitudinal axis (A), in a second rotational direction opposite said first rotational direction, such that each of the outer wings (204) of the connector engage one of the locking slots (6), the locking slots (6) including a mechanical stop (62) for stopping rotation of the connector in a second angular position and
an abutment surface (60) for preventing the connector from moving back in the distal direction.

2. Adaptor (1) according to the preceding claim, wherein the inner ring (3) has inwardly protruding lugs (30) for abutting against the distal tip (102).

3. Adaptor (1) according to the preceding claim, wherein the inner ring (3) includes a distal side (33) having a tapered or frustroconical shape.

4. Adaptor (1) according to any of the preceding claims, wherein the locking slots (6) are through-openings extending through the lateral wall (2) of the adaptor (1).

5. Adaptor (1) according to any of the preceding claims, wherein the first angular position and the second angular position are separated by an angle (α) comprised between 40° and 90°, preferably between 50° and 80°, and even more preferably by an angle of 65°.

6. Adaptor (1) according to any of the preceding claims, wherein the inner bulges (4) include a second blocking surface (45) for blocking rotation of the connector in the second rotational direction as long as the connector is being slid inside the adaptor, the second blocking surface (45) being configured to allow rotation of the connector in the second rotational direction when the connector is in an inserted position.

7. Adaptor (1) according to any of the preceding claims, wherein the adaptor (1) includes a tapered portion (24) extending between the opened distal end (23) of the adaptor (1) and the inner cavity (40).

8. Adaptor (1) according to any of the preceding claims, wherein the inner bulges (4) have a flat distal surface (41).

9. Adaptor (1) according to any of the preceding claims, wherein the two inner bulges (4), and respectively the two locking slots (6), are diametrically opposite.

10. Tip cap assembly (202) configured to be connected to an adaptor (1) according to any of the preceding claims, wherein the tip cap assembly (202) has two outwardly protruding wings (204) configured to engage the locking slots (6) of the adaptor (1) for removably securing the tip cap assembly (202) to the adaptor (1).

11. Drug delivery device (300) including a medical container (100) having a distal tip (102) longitudinally extending from a barrel (106) defining a reservoir for a medical product, an adaptor (1) according to any of claims 1-9 configured to be mounted onto the distal tip (102), and a connector, such as a needle hub (200) or a tip cap assembly (202), configured to be removably secured to the distal tip (102) by means of said adaptor (1).

12. Drug delivery device (300) according to the preceding claim, wherein the connector and the inner cavity (40) of the adaptor (1) are complementarily shaped.

13. Drug delivery device (300) according to claim 11 or 12, wherein the proximal abutment surface (60) of each of the locking slots (6) abuts against at least 50%, preferably between 50% and 90%, of a distal side of the corresponding outwardly protruding wing (204) when the connector is in the second angular position.

14. Method for manufacturing an adaptor (1) according to any of claims 1-9, wherein the method is devoid of the use of a rotating core pin.
